# EUROPEAN PATENT APPLICATION

(11) **EP 4 080 216 A1**
(43) Date of publication of application: **26.10.2022**
(21) Application number: 21170193.3
(22) Date of filing: 23.04.2021
(51) Int. Cl.: G01N 33/569, A61K 39/00

(54) **METHODS DETERMINING THE POTENTIAL OF DRUGS FOR TREATING BACTERIAL INFECTIONS AND COMPOSITION FOR TREATING BACTERIAL INFECTIONS**

(71) Applicant: Institute of Science and Technology Austria, 3400 Klosterneuburg (AT); VALANX Biotech GmbH, 3400 Klosterneuburg (AT)
(72) Inventor: Tomasek, Kathrin, 3400 Klosterneuburg (AT); Guet, Calin, 3400 Klosterneuburg (AT); Sixt, Michael, 3400 Klosterneuburg (AT); Lukesch, Michael, 3400 Klosterneuburg (AT)
(74) Representative: Grund, Martin

(57) **Abstract**

The present invention relates to an *in vitro* method for testing a drug for the treatment of bacterial infections, wherein the method is characterized by determining the potential of the drug to inhibit binding of type I piliated gram-negative bacteria to CD14.

The present invention also relates to a CD14 antibody for use in the treatment of urinary tract infections as well as a combination of a CD14 antibody with antibiotics, mannose and/or mannoside. The present invention also relates to a CD14 antibody for use in the treatment of persistent and/or recurrent urinary tract infections.

## Description

### FIELD OF THE INVENTION

The present invention relates to an *in vitro* method for testing a drug for the treatment of bacterial infections, wherein the method is characterized by determining the potential of the drug to inhibit binding of type I piliated gram-negative bacteria to CD14.

The present invention also relates to a CD14 antibody for use in the treatment of urinary tract infections as well as a combination of a CD14 antibody with antibiotics, mannose and/or mannoside. The present invention also relates to a CD14 antibody for use in the treatment of persistent and/or recurrent urinary tract infections.

### INTRODUCTION

Bacterial infections are a major cause of morbidity and mortality in humans. It is estimated that antibiotic-resistant bacterial infections alone contribute to 2.8 million cases in the US each year resulting in 35,000 deaths which also imposes a significant burden on the healthcare system. Hence, the number of cases and deaths for any bacterial infection are significantly higher. Often, the treatment of bacterial infections does not lead to the desired result, the bacterial infections become persistent or recurrent and the patients may experience undesired and even serious side effects from prolonged treatment with for example, antibiotics.

Bacteria cause infections by various mechanisms, for example by secreting or excreting toxins, by producing toxins internally, which are released when the bacteria disintegrate, or by inducing sensitivity to their antigenic properties. All human organs are susceptible to bacterial infection. Urinary tract infections, (neonatal) meningitis and gastrointestinal infections such as salmonellosis *inter alia* are examples of bacterial infections; bacterial infections are also believed to be the cause of Crohn's Disease and ulcerative colitis. Urinary tract infections, for example, are one of the commonest bacterial infections worldwide, caused mainly by uropathogenic *E. coli* (UPEC), with 150 million cases per year worldwide leading to up to 6 billion US dollars spent on treatment. The high recurrence rate of these infections can be partially accounted for due to the limited response of the adaptive immune system, especially during cystitis. In any case of bacterial infection, upon infection, dendritic cells, although part of the innate immune system, migrate from the site of infection to lymph nodes to activate the adaptive immune response.

CD14, a surface molecule belonging to the cluster of differentiation superfamily, is known to be expressed on various immune cells including dendritic cells and macrophages. The inventors identified CD14 on dendritic cells as hitherto unknown receptor for the FimH protein. FimH is part of type I pili of gram-negative bacteria. Binding of type I pili of gram-negative bacteria to CD14, precisely the interaction between CD14 and FimH leads to severe changes in the behavior of dendritic cells and therefore impacts activation and proliferation of adaptive immune cells.

It is widely known that treatment of bacterial infection does not always lead to the improvement of the patients' condition. In addition, patients are prone to suffer from side effects from the treatment which is even worse when the treatment does not yield in the desired effect of fighting the infection. Therefore, there is a great need for methods capable of determining the potential of the drug to inhibit binding of type I piliated gram-negative bacteria to CD14 in *vitro.* There is also the need to treat specific types of infections such as urinary tract infections by targeted CD14 therapy, alone or in combination with other agents such as antibiotics, mannose and/or mannoside. It would be desirable if such methods identified agents which would lead to adequate treatments of infections and therefore, be administered in a cost-effective fashion. Furthermore, novel approaches are required to combat bacterial infection in a sustainable and safe manner.

### SUMMARY OF THE INVENTION

In one aspect, a method for testing a drug for the treatment of bacterial infections is provided, wherein the method is characterized by determining the potential of the drug to inhibit binding of type I piliated gram-negative bacteria to CD14 *in vitro* is claimed. In one embodiment, the type I piliated negative gram-negative bacteria express FimH. In another embodiment, the method comprises the steps of: a) coupling magnetic beads to a CD14 protein, b) providing type I piliated gram-negative bacteria having a fluorescent marker stably integrated in their DNA or, alternatively, visualizing the DNA by a fluorescent marker which is added after step f), c) adding type I piliated gram-negative bacteria expressing FimH to the CD14-coupled magnetic beads, d) incubating said type I piliated gram-negative bacteria with the CD14-coupled magnetic beads and adding a drug or a compound, e) removing any unbound bacteria from the beads, f) optionally fixing the bound bacteria with paraformaldehyde, g) analyzing the type I piliated gram-negative bacteria bound to the CD14-coupled magnetic beads by fluorescence microscopy or flow cytometry, wherein a decrease in the at least one fluorescent marker in the presence of the drug or the compound indicates that the drug or the compound inhibits binding of FimH to CD14. In yet another embodiment, D-mannose, mannoside and/or a CD14 antibody inhibit binding of gram-negative bacteria expressing FimH to CD14 coupled magnetic beads. In even another embodiment, the method comprises the steps of: a) providing dendritic cells, b) infecting said dendritic cells with type I piliated gram-negative bacteria expressing FimH, c) adding a drug or a compound to the dendritic cells, d) adding an antibiotic to kill the bacteria that have not been engulfed by said dendritic cells, e) removing non-adherent dendritic cells after a defined time period, thereby f) obtaining infected adherent dendritic cells only, g) adding a fluorescent dye to said infected adherent dendritic cells, h) quantifying the fluorescent signal by a fluorescence plate reader, wherein a decrease in the at least one fluorescent signal in the presence of the drug or the compound indicates that the drug or the compound decreases adhesion of said infected dendritic cells. In another embodiment, the dendritic cells are infected, i.e., activated dendritic cells. In yet another embodiment, the dendritic cells are infected, i.e., activated murine dendritic cells. In another embodiment, the drug or compound leads to decreased adhesion of the dendritic cells compared to untreated dendritic cells. In yet another embodiment, the type I piliated gram-negative bacteria are selected from *Escherichia coli* and/or *Klebsiella pneumoniae, or Salmonella spp.* In even another embodiment, FimH harbors a R98A, or T99A, or Y48A mutation or any combination thereof. In another embodiment, the drug or the compound is selected from the group consisting of D-mannose, small mannoside, CD14 antibody, CD14 inhibitor or an antibiotic. In yet another embodiment, the method is used in determining the potential of a drug for the treatment of urinary tract infections caused by type I piliated gram-negative bacteria. In another embodiment, the method is used in determining the potential of a drug for the treatment of neonatal meningitis, inflammatory bowel diseases such as Crohn's disease, or ulcerative colitis, or gastrointestinal disorders or gastrointestinal diseases caused by type I piliated gram-negative bacteria.

In another aspect, the method is used to inform the selection of drugs in the treatment of urinary tract infections.

In yet another aspect, a CD14 antibody is used for the treatment of urinary tract infections. In one embodiment, a combination of a CD14 antibody and antibiotics, or CD14 antibody and D-mannose, or CD14 antibody and mannoside, or CD14 antibody, antibiotics, and D-mannose or mannoside is used for the treatment of urinary tract infections. In another embodiment, a CD14 antibody is used for the treatment of persistent and/or recurrent urinary tract infections. In one embodiment, the CD-14 antibody for use in the treatment of urinary tract infections, or persistent and/or recurrent urinary tract infections is Atibuclimab.

### BRIEF DESCRIPTION OF THE FIGURES

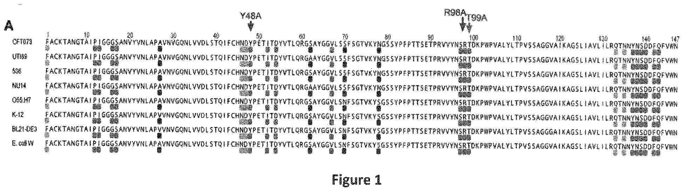

**Figure 1****:** Selection of a FimH sequence alignment of FimH among several pathogenic and non-pathogenic *E. coli* strains. Amino acids which are responsible for binding to CD14 are highly conserved.

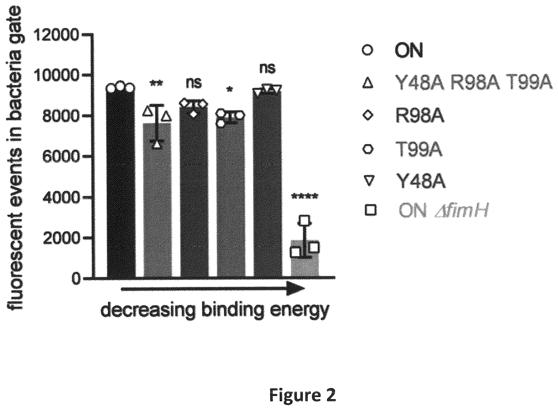

**Figure 2****:** Bar diagram of the number of fluorescent bacteria bound to CD14-coupled beads in flow cytometry experiment of four FimH amino acid mutants (Y48A/R98A/T99A, R98A, T99A, Y48A) ("bead assay"). Surprisingly, all four FimH mutants are still able to bind to CD14-coupled beads and mainly the triple mutant only showed decreased binding. This suggests that not only these individual amino acids drive binding to CD14, but most likely several other amino acids and the supporting secondary structure of the FimH protein is involved. ON bacterial mutants served as positive control (high binding) and ON bacterial mutants with a deletion of FimH served as negative control (low binding).

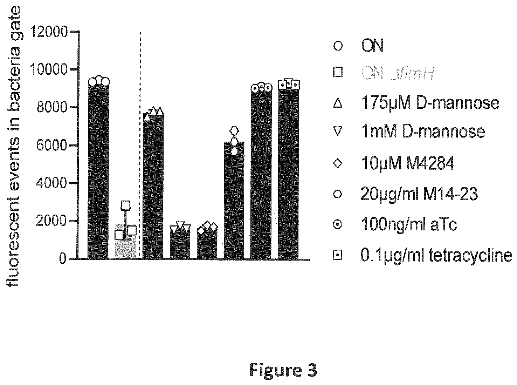

**Figure 3****:** Bar diagram of the number of fluorescent bacteria bound to CD14-coupled beads in flow cytometry experiment using D-mannose, M4284, M14-23, aTc and tetracycline treatments at different concentrations ("bead assay"). D-mannose at 1 mM and the small mannoside M4284 at 10 µM prevented binding of bacteria to CD14. The anti CD14 antibody at 20 µg/ml prevented binding partially. ON bacterial mutants served as positive control (high binding) and ON bacterial mutants with a deletion of FimH served as negative control (low binding).

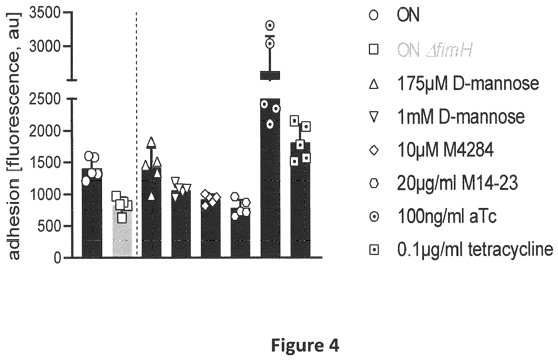

**Figure 4****:** Bar diagram of the fluorescent signal determined from adherent dendritic cells by a fluorescent plate reader experiment ("adhesion assay"). Treatments with 1 mM of D-mannose, 10 µM of M4284 and 20 µg/ml M14-23, respectively resulted in decreased adhesion of dendritic cells and would therefore, translate into successful treatments.
Surprisingly, treatment with the tetracycline-analog anhydrotetracycline or tetracycline increased adhesion even further, demonstrating that antibiotics in this very example would be a suboptimal treatment choice.

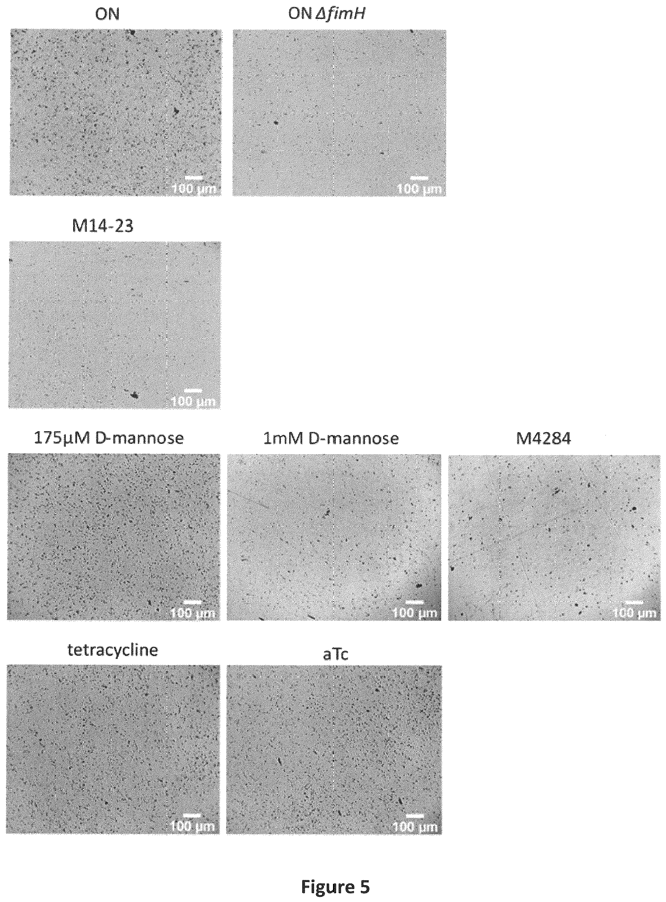

**Figure 5****:** Brightfield images of adherent dendritic cells ("adhesion assay"). Treatments with 1 mM of D-mannose, 10 µM of M4284 and 20 µg/ml M14-23, respectively resulted in decreased adhesion of dendritic cells and would therefore, translate into successful treatments. Surprisingly, treatment with the tetracycline-analog anhydrotetracycline or tetracycline increased adhesion even further, demonstrating that antibiotics in this very example would be a suboptimal treatment choice. ON bacterial mutants served as positive control (increased adhesion of dendritic cells) and ON bacterial mutants with a deletion of FimH served as negative control (decreased adhesion of dendritic cells).

### DETAILED DESCRIPTION OF THE INVENTION

### Definitions

*"**In vitro"*** refers to studies and experiments, which are performed with microorganisms such as bacteria on cells and in some cases, biological molecules such as antibodies outside their normal biological context, i.e., a living organism in the context of the present invention. Therefore, *in vitro* experiments in accordance with the present invention are performed for example in a test tube or a culture dish.

*"**Potential**"* or ***"determining the potential"*** refers to the ability of a drug or compound such as a CD14 antibody, CD14 inhibitor, CD14 binding molecule, D-mannose, small mannoside, antibiotic etc. taking the example of urinary tract infections to fight infections in the context of the present invention. Therefore, a drug or compound having the potential to target the FimH-CD14 interaction is likely to be an efficacious compound with regards to its ability to counteract and ultimately, fight bacterial infections. A drug or compound has potential according to the present invention, if e.g., the drug or compound decreases the binding of FimH to CD14 or even completely inhibits the binding of FimH to CD14, compared to the negative control, respectively. A drug or compound also has potential according to the present invention, if e.g., the drug or compound decreases the adhesion of dendritic cells or even completely abolishes the adhesion of dendritic cells, compared to the negative control, respectively. Therefore, a FimH antagonist or FimH inhibitor is likely to be an efficacious compound with regards to its ability to counteract and ultimately, fight bacterial infections by for example decreasing or completely inhibiting the binding of FimH to CD14 and/or decreasing the adhesion of for example dendritic cells.

***"CD14 binding molecule"*** refers to a molecule that is capable of disrupting the binding of an interaction or binding partner such as FimH to CD14, or any molecule that binds with a sufficiently high affinity to FimH. Said molecule can be used for the treatment of bacterial infections in which the inhibition or disruption of type I piliated gram-negative bacteria binding via FimH to CD14 is desirable. Preferably, the CD14 binding molecule binds with high affinity to R98, and/or T99, and/or Y48, or S17, and/or 113, and/or P12 of FimH. The CD14 binding molecule can also bind with high affinity to e.g., Y48, and/or 152 and/orY137 of FimH's tyrosine gate. Alternatively, the CD14 binding molecule can also bind to e.g. P1, and/or N46, and/or D47, and/or D54, and/or Q133, and/or D135, and/or D140, and/or F142 of FimH's mannose-binding pocket. The CD14 binding molecule can also bind to any combination of the above disclosed amino acids, such as R98, any position(s) of FimH's tyrosine gate and any position(s) of the mannose-binding pocket. Alternatively, the CD14 binding molecule binds with high affinity to any of S17, and/or 113, and/or P12, and/or D54, and/or F142, and/or N138, and/or V67, and/or D140, and/or Y137, and/or G16 of FimH. Preferably, the CD14 binding molecule binds with high affinity to S17, and/or 113, and or P12 of FimH. Alternatively, the CD14 binding molecule can also bind to e.g., D54, and/or F142, and/or N138, and/or V67, and/or D140, and/or Y137, and/or G16 of FimH. The CD14 binding molecule can also bind to any combination of the above disclosed amino acids, such as S17 and any other position(s) of FimH. Suitable CD14 binding molecules of the present invention are for example D-mannose or mannosides such as M4284 that interfere with the binding of FimH with CD14. In some instances, CD14 binding molecules are CD14 inhibitors.

***"CD14 antibody"*** refers to an antibody that targets and therefore, binds to CD14. By binding to CD14, the FimH-CD14 interaction is at least weakened and therefore, at least partially, inhibited. Preferably, the FimH-CD14 interaction is completely inhibited. A CD14 antibody according to the invention can be a monoclonal antibody, a polyclonal antibody, a chimeric antibody, a humanized antibody, or a synthetic antibody. The CD14 antibody or fragment thereof can be from any species such as mouse, rabbit, hamster, rabbit, goat, sheep, chicken, human. The CD14 antibody can be a humanized antibody. The CD14 antibody or fragment thereof can be a chimeric antibody according to the invention. The CD14 antibody can be of any isotype (e.g., IgG, IgE, IgM, IgD, and IgA), class (e.g., IgG1, IgG2, IgG3, IgG4, IgA1 and IgA2) or derivative thereof. The CD14 antibody or fragment thereof according to the invention can be a Fab, F(ab2)', F(ab)2' or scFv. Exemplary antibodies are the human chimeric antibody Atibuclimab and the murine antibody M14-23.

***"CD14 inhibitor"*** refers to a molecule or compound that tends to nullify the action of another, or in some instances that blocks the ability of a given chemical or interaction or binding partner such as FimH to bind to CD14 or other interacting molecule, thereby preventing a biological response according to the present invention. Said inhibitor can be used for the treatment of bacterial infections in which the inhibition or disruption of type I piliated gram-negative bacteria binding via FimH to CD14 is desirable. Preferably, the CD14 inhibitor binds with high affinity to R98, and/or T99, and/or Y48, or S17, and/or 113, and/or P12 of FimH. The CD14 inhibitor can also bind with high affinity to e.g., Y48, and/or 152 and/or Y137 of FimH's tyrosine gate. Alternatively, the CD14 inhibitor can also bind to e.g. P1, and/or N46, and/or D47, and/or D54, and/or Q133, and/or D135, and/or D140, and/or F142 of FimH's mannose-binding pocket. The CD14 inhibitor can also bind to any combination of the above disclosed amino acids, such as R98, any position(s) of FimH's tyrosine gate and any position(s) of the mannose-binding pocket. Alternatively, the CD14 inhibitor binds with high affinity to any of S17, and/or I13, and/or P12, and/or D54, and/or F142, and/or N128N138, and/or V67, and/or D140, and/or Y137, and/or G16 of FimH. Preferably, the CD14 inhibitor binds with high affinity to S17, and/or 113, and or P12 of FimH. Alternatively, the CD14 inhibitor can also bind to e.g., D54, and/or F142, and/or N128N138, and/or V67, and/or D140, and/or Y137, and/or G16 of FimH. The CD14 inhibitor can also bind to any combination of the above disclosed amino acids, such as S17 and any other position(s) of FimH. Inhibitors are not limited per se to a specific type of compound, and may include peptides, organic or inorganic compounds (for example, small molecules). The term "inhibitor" is generally synonymous with "antagonist". Suitable inhibitors of the present invention are for example D-mannose or mannosides such as M4284 that interfere with the binding of FimH with CD14.

***"D-mannose"*** is a C2-epimer of glucose and therefore, a monosaccharide. D-mannose is used as a supplement for the prevention and treatment of bacterial infections caused by *E.coli* such as urinary tract infections. D-mannose in the context of the present invention targets and interferes with the binding of FimH to CD14.

***"Mannoside",*** also small mannoside refers to any glycoside of mannose. Said mannoside can be further modified (e.g., with one or more additional functional groups such as a phenyl group) and be e.g., a dimannoside, hexamannoside, monomannoside, oligomannoside, or trimannoside. One potent mannoside according to the invention is for example M4284. Mannosides in the context of the present invention such as M4284 target and interfere with the binding of FimH to CD14.

***"Persistent"*** refers to a bacterial infection and/or medical condition that continues to exist or occur over a prolonged period in the context of the present invention. Persistent may also imply that various treatments have been tried and tested, yet the bacterial infection and/or medical infection continued to exist. Sometimes, persistent infections are also called chronic infections.

***"Recurrent"*** refers to a bacterial infection and/or medical condition that follows resolution of an earlier episode, usually after appropriate treatment. Recurrent infections include relapses, i.e., symptomatic recurrent infections with the same organism following the adequate therapy and reinfection, i.e., recurrent infections with previously isolated bacteria after treatment or a recurrent infection caused by a second e.g., bacterial isolate.

In one aspect, a method for testing a drug for the treatment of bacterial infections is provided, wherein the method is characterized by determining the potential of the drug to inhibit binding of type I piliated gram-negative bacteria to CD14 *in vitro* is claimed.

### Detailed description

In one embodiment, the type I piliated negative gram-negative bacteria express FimH.

In another embodiment, the method comprises the steps of: a) coupling magnetic beads to a CD14 protein, b) providing type I piliated gram-negative bacteria having a fluorescent marker stably integrated in their DNA or, alternatively, visualizing the DNA by a fluorescent marker which is added after step f), c) adding type I piliated gram-negative bacteria expressing FimH to the CD14-coupled magnetic beads, d) incubating said type I piliated gram-negative bacteria with the CD14-coupled magnetic beads and adding a drug or a compound, e) removing any unbound bacteria from the beads, f) optionally fixing the bound bacteria with paraformaldehyde, g) analyzing the type I piliated gram-negative bacteria bound to the CD14-coupled magnetic beads by fluorescence microscopy or flow cytometry, wherein a decrease in the at least one fluorescent marker in the presence of the drug or the compound indicates that the drug or the compound inhibits binding of FimH to CD14. In one embodiment, the CD14 protein is a recombinant chimeric protein. In a preferred embodiment, the CD14 protein comprises the murine N-terminal of CD14 which is bound to protein A on the magnetic beads via the Fc tail. In another embodiment, the magnetic beads are pre-coated with protein A. In one preferred embodiment, a fluorescent marker is stably integrated into the DNA of the bacteria. The stable integration of a fluorescent marker into the bacteria DNA is performed by any conventional means known to the skilled person such as by transforming the bacteria with a conditional-replication, integration, and modular (CRIM) plasmid. In another embodiment, the fluorescent marker is transiently integrated into the DNA of the bacteria. The transient integration of a fluorescent marker into the bacteria DNA is performed by any conventional means known to the skilled person such as by transforming the bacteria with a plasmid harbouring the fluorescent marker. In another embodiment, the DNA is visualized by a fluorescent marker added after step f). In a preferred embodiment, the fluorescent marker added after step f) is a fluorescent dye. In the latter embodiment, the fluorescent marker or fluorescent dye is thiazole orange, Hoechst 3342, Hoechst 3358, or carboxyfluorescein diacetate (CFDA) and its derivatives. In one embodiment, the infection causing type I piliated gram-negative bacteria are causing neonatal meningitis. In another embodiment, the infection causing type I piliated gram-negative bacteria are causing inflammatory bowel diseases such as Crohn's disease or ulcerative colitis. In one embodiment, the infection causing type I piliated gram-negative bacteria causes Crohn's disease. In another embodiment, the infection causing type I piliated gram-negative bacteria causes ulcerative colitis. In yet another embodiment, the infection causing type I piliated gram-negative bacteria causes gastrointestinal disorders or gastrointestinal diseases such as salmonellosis. In a preferred embodiment, the infection causing type I piliated gram-negative bacteria cause urinary tract infections. In a particularly preferred embodiment, the type I piliated gram-negative bacteria causing urinary tract infections are uropathogenic *E. coli.* In one embodiment, the incubation of the type I piliated gram-negative bacteria with the CD14-coupled magnetic beads and the added drug or compound lasts between ten minutes and six hours. In one embodiment, the incubation takes between 30 minutes and three hours. In a particularly preferred embodiment, the incubation takes between 45 and 90 minutes. In one embodiment, the added drug or compound is an inhibitor, antibody or antibiotic. In one preferred embodiment, the added drug or compound is D-mannose, small mannoside, CD14 antibody, CD14 inhibitor or an antibiotic. In a particularly preferred embodiment, the added drug or compound is D-mannose or the small mannoside M4284. In one embodiment, the bacteria are pre-incubated with the D-mannose, followed by adding the bacteria with the D-mannose to the CD14-coupled magnetic beads. In one embodiment, the pre-incubation lasts between five minutes and 24 hours. In a preferred embodiment, the pre-incubation lasts between one and four hours. In another embodiment, the bacteria are pre-incubated with the small mannoside followed by adding the bacteria with the small mannoside to the CD14-coupled magnetic beads. In a preferred embodiment, the pre-incubation lasts between one and four hours. In yet another embodiment, the CD14 antibody is pre-incubated with the CD14-coupled magnetic beads, followed by adding the bacteria. In a preferred embodiment, the pre-incubation lasts between one and four hours. In one embodiment, the unbound bacteria are removed from the beads by washing. In one embodiment, the unbound bacteria are removed from the beads by washing with PBS-T. In a preferred embodiment, the unbound bacteria are removed from the beads by washing with M9 glycerol medium comprising Tween^{®}20, calcium and magnesium ions as described in Tomasek et al. 2018 J Biotechnol. In one embodiment, the bound bacteria are fixed by a fixing agent. The fixing agent can be e.g., -20°C cold acetone, followed by graded ethanol from a low to a high concentration followed by PBS, glyoxal or paraformaldehyde. In a preferred embodiment, the bound bacteria are fixed with paraformaldehyde at 0.05%-5% paraformaldehyde. In a particularly preferred embodiment, the bacteria are fixed with 0.5% paraformaldehyde. In another embodiment, the bacteria are not fixed at all by a fixing agent. In one embodiment, the type I piliated gram-negative bacteria bound to the CD14-coupled magnetic beads are analyzed by fluorescence microscopy. In a preferred embodiment, the type I piliated gram-negative bacteria bound to the CD14-coupled magnetic beads are analyzed by flow cytometry. In a preferred embodiment, a decrease in the at least one fluorescent marker in the presence of the drug or the compound indicates that the drug or the compound inhibits binding of FimH to CD14. In another embodiment, an increase in the at least one fluorescent marker in the presence of the drug or the compound indicates that the drug or the compound does not inhibit binding of FimH to CD14.

In yet another embodiment, D-mannose, mannoside and/or a CD14 antibody inhibit binding of gram-negative bacteria expressing FimH to CD14 coupled magnetic beads. In one embodiment, CD14 binding molecules or CD14 inhibitors are used. In one embodiment, D-mannose is used. In a preferred embodiment, the D-mannose is used at a concentration higher than 175 µM. In another embodiment, mannoside is used. In a preferred embodiment, the mannoside M4284 is used. In another embodiment, M4284 is used at a concentration of 1 µM to 100 µM. In a particularly preferred embodiment, M4284 is used at a concentration of 5 µM to 20 µM. In one embodiment, a combination of D-mannose and mannoside is used. In yet another embodiment, a CD14 antibody is used. In one embodiment, the CD14 antibody is M14-23 or Atibuclimab. In one embodiment, about 5 to 50 µg/ml M14-23 is used. In a preferred embodiment, about 10 to 30 µg/ml M14-23 is used. In an even more preferred embodiment, 20 µg/ml M14-23 is used. In yet another embodiment, D-mannose, mannoside, and CD14 antibody are used.

In even another embodiment, the method comprises the steps of: a) providing dendritic cells, b) infecting said dendritic cells with type I piliated gram-negative bacteria expressing FimH, c) adding a drug or a compound to the dendritic cells, d) adding an antibiotic to kill the bacteria that have not been engulfed by said dendritic cells, e) removing non-adherent dendritic cells after a defined time period, thereby f) obtaining infected adherent dendritic cells only, g) adding a fluorescent dye to said infected adherent dendritic cells, h) quantifying the fluorescent signal by a fluorescence plate reader, wherein a decrease in the at least one fluorescent signal in the presence of the drug or the compound indicates that the drug or the compound decreases adhesion of said infected dendritic cells. In one embodiment, the dendritic cells are dendritic cells from any mammalian species such as human, primate, mouse, cow, pig, dog, goat, rabbit, rat and cow. In one embodiment, the dendritic cells are human or murine dendritic cells. In another embodiment, the dendritic cells are established dendritic cell lines. In another embodiment, the dendritic cells are human dendritic cell lines such as Thp-1, HL-60, MUTZ-3, U937, KG-1 and PDMC05. In one embodiment, the dendritic cells are cultured in regular cell culture dishes or cell culture plates known to the skilled person in the art. In another embodiment, the cell culture dishes or cell culture plates are treated with different extracellular matrix components such as fibronectin or fibrinogen or combinations thereof. In another embodiment, genetically modified dendritic cells are used. In a preferred embodiment, the genetically modified dendritic cells express a fluorescent marker. In a particularly preferred embodiment, the genetically modified dendritic cells have a fluorescently labelled actin cytoskeleton. In a particularly preferred embodiment, the genetically modified dendritic cells express LifeAct^{®}-GFP and therefore, have a fluorescently labelled actin cytoskeleton. In one embodiment, the dendritic cells are infected with the bacteria at a multiplicity of infection of 1-100 (1-100 bacteria per 1 dendritic cell). The skilled person is aware that infected dendritic cells are activated dendritic cells. In another embodiment, the dendritic cells are infected with the bacteria at a multiplicity of infection of 1-30 (1-30 bacteria per 1 dendritic cell). In a particularly preferred embodiment, the dendritic cells are infected with the bacteria at a multiplicity of infection of 10 (10 bacteria per 1 dendritic cell). In one embodiment, the infection causing type I piliated gram-negative bacteria are causing neonatal meningitis. In another embodiment, the infection causing type I piliated gram-negative bacteria are causing inflammatory bowel diseases such as Crohn's disease or ulcerative colitis. In one embodiment, the infection causing type I piliated gram-negative bacteria causes Crohn's disease. In another embodiment, the infection causing type I piliated gram-negative bacteria causes ulcerative colitis. In yet another embodiment, the infection causing type I piliated gram-negative bacteria causes gastrointestinal disorders or gastrointestinal diseases such as salmonellosis. In a preferred embodiment, the infection causing type I piliated gram-negative bacteria cause urinary tract infections. In a particularly preferred embodiment, the type I piliated gram-negative bacteria causing urinary tract infections are uropathogenic *E. coli.* In one embodiment, the added drug or compound is a CD14 inhibitor, CD14 antibody, CD14 binding molecule or an antibiotic. In one preferred embodiment, the added drug or compound is D-mannose, small mannoside, CD14 antibody, CD14 inhibitor or an antibiotic. In a particularly preferred embodiment, the added drug or compound is the small mannoside M4284 or a CD14 antibody. In one embodiment, the incubation of the dendritic cells which engulfed the bacteria and the added drug or compound lasts between ten minutes and 72 hours. In one embodiment, the incubation takes between two and 48 hours. In a particularly preferred embodiment, the incubation takes between 18 and 20 hours. In one embodiment, the bacteria are pre-incubated with the D-mannose, followed by adding the bacteria with the D-mannose to the dendritic cells. In one embodiment, the pre-incubation lasts between five minutes and 24 hours. In a preferred embodiment, the pre-incubation lasts between one and four hours. In another embodiment, the bacteria are pre-incubated with the small mannoside followed by adding the bacteria with the small mannoside to the dendritic cells. In a preferred embodiment, the pre-incubation lasts between one and four hours. In yet another embodiment, the CD14 antibody is pre-incubated with the dendritic cells, followed by adding the bacteria. In one embodiment, the pre-incubation lasts between five minutes and four hours. In a preferred embodiment, the pre-incubation lasts between five and thirty minutes. In an even more preferred embodiment, the pre-incubation lasts twenty minutes. In even another embodiment, the CD14 antibody and the bacteria are added to the dendritic cells at substantially the same or the same time. In yet another embodiment, the bacteria are added to the dendritic cells and the CD14 antibody is added about five minutes to four hours later to the bacteria and dendritic cells. In a preferred antibody, the bacteria are added to the dendritic cells and the CD14 antibody is added about five to thirty minutes later. In one embodiment, an antibiotic is added to kill the bacteria that have not been engulfed by said dendritic cells. In one embodiment, a combination of multiple antibiotics is added. In another embodiment, gentamicin or kanamycin is added. In a preferred embodiment, gentamicin is added. In one embodiment, the antibiotic is added 15 minutes to eight hours post-infection. In another embodiment, the antibiotic is added 30 minutes to three hours post-infection. In a preferred embodiment, the antibiotic gentamicin is added one hour post-infection. In one embodiment, the non-adherent dendritic cells are removed after between two and 48 hours. In a particularly preferred embodiment, the non-adherent dendritic cells are removed after between 18 and 20 hours. In one embodiment, the adherent dendritic cells are obtained. In one embodiment, the adherent dendritic cells are subjected to fixation, followed by adding a dye such as trypan blue or crystal violet. In one embodiment, crystal violet is subsequently extracted from the adherent dendritic cells by acetic lysis. In one embodiment, the cells dyed by trypan blue or crystal violet are analysed on a colorimetric plate reader or by visual inspection. In another embodiment, the adherent dendritic cells are not subjected to fixation and a fluorescent dye such as calcein AM, carboxyfluorescein diacetate (CFDA), 5-TAMRA, Oregon Green^{™}, Hoechst 3342, Hoechst 33258, or DAPI is added to said cells. In a preferred embodiment, the added fluorescent dye is Hoechst 3342. In one embodiment, the dendritic cells are analysed by flow cytometry followed by harvesting, staining and normalizing said dendritic cells to obtain the amount of adherent cells per sample, thereby quantifying said dendritic cells. In another embodiment, fluorescence microscopy is used to analyse the amount of adherent cells, thereby quantifying the dendritic cells in a defined field of view. In yet another embodiment, bright field microscopy is used to analyse the amount of adherent cells, thereby quantifying the dendritic cells in a defined field of view. In even another embodiment, one or more adhesion receptors are detected on adherent cells to analyse the amount of adherent cells. A suitable adhesion receptor is CD45 *inter alia.* The detection of adherent cells by adhesion receptors can be determined by e.g. antibody detection and for example a CD45 antibody that is labelled by e.g. a fluorescent or colorimetric tag or can be detected by a secondary fluorescent or colorimetric antibody. In even another embodiment, a co-stimulatory molecule receptor such as MHCI class II can serve as an activation marker of dendritic cells. The detection of said activated dendritic cells by the detection of MHC class II can be performed by e.g., antibody detection and for example a MHC class II antibody that is labelled by e.g. a fluorescent or colorimetric tag or can be detected by a secondary fluorescent or colorimetric antibody. The resulting signal can be detected by various means known to the skilled person such as fluorescence microscopy or flow cytometry. Co-stimulatory molecules such as CD40, CD80 and CD86 do not appear to be suitable targets in certain instances since these receptors are downregulated on dendritic cells after stimulation with type I piliated *E. coli.* In one preferred embodiment, the fluorescent signal is quantified by a fluorescence plate reader, wherein a decrease in the at least one fluorescent signal in the presence of the drug or the compound indicates that the drug or the compound decreases adhesion of said infected dendritic cells. In another embodiment, the fluorescent signal is quantified by a fluorescence plate reader and additionally, bright field microscopy is also used to analyse the amount of adherent cells. In yet another embodiment, fluorescence and/or brightfield microscopy can be used to obtain another read-out to confirm that adhesion increases or decreases, indeed.

In one embodiment, the dendritic cells are infected, i.e., activated murine dendritic cells. In one embodiment, the dendritic cells are activated by the addition of the bacteria or by the addition of LPS. In one embodiment, the LPS is added at a concentration of 50-500 ng/ml. In a preferred embodiment, the LPS is added at a concentration of 100-300 ng/ml. In a particularly preferred embodiment, the LPS is added at a concentration of 200 ng/ml. In a preferred embodiment, the dendritic cells are infected, i.e., activated by the addition of the bacteria. In one embodiment, the murine dendritic cells are generated from Hoxb8 progenitor cells. In another embodiment, the murine dendritic cells are prepared from immortalized progenitor cells. In one embodiment, the immortalized progenitor cells are Hoxb8 progenitor cells. In another embodiment, the murine dendritic cells are prepared from monocytes from the blood or bone marrow. In yet another embodiment, the murine dendritic cells are selected from murine dendritic cell lines or murine dendritic cell-like cell lines such as D1, Raw264.7 or JASIII. In a particularly preferred embodiment, the murine dendritic cells are generated from Hoxb8 progenitor cells.

In another embodiment, the drug or compound leads to decreased adhesion of the dendritic cells compared to untreated dendritic cells.

In yet another embodiment, the type I piliated gram-negative bacteria are selected from *E. coli* and/or *Klebsiella pneumoniae, or Salmonella spp.* In one embodiment, *E. coli* cause neonatal meningitis, inflammatory bowel diseases such as Crohn's disease, or ulcerative colitis or gastrointestinal diseases or disorders such as salmonellosis, or urinary tract infections. In another embodiment, the *Klebsiella pneumoniae* cause neonatal meningitis, inflammatory bowel diseases such as Crohn's disease, or ulcerative colitis, gastrointestinal disorders or gastrointestinal diseases or urinary tract infections. In another embodiment, *Salmonella spp.* is selected from the group consisting of all nontyphoid serotypes of the *Salmonella* genus except for *Salmonella typhi* and *Salmonella paratyphi* A, B and C and causes salmonellosis. In one particularly preferred embodiment, the type I piliated gram-negative bacteria are selected from *E. coli* which cause urinary tract infections, i.e., UPEC. In another particularly preferred embodiment, the type I piliated gram-negative bacteria are selected from *Klebsiella pneumoniae* which cause urinary tract infections. In another particularly preferred embodiment, the type I piliated gram-negative bacteria are selected from *E. coli* and *Klebsiella pneumoniae* which cause urinary tract infections.

In another embodiment, FimH habours a mutation at position R98, or T99, or Y48 or any combination thereof. In one embodiment, FimH harbours either a mutation at position R98, or T99, or at Y48 alone. In another embodiment, FimH harbours a triple mutation, i.e., a mutation at position R98, T99, and Y48. In yet another embodiment, FimH harbours a mutation at position R98 and T99 mutation but does not harbour a mutation at position Y48. In even another embodiment, FimH harbour a mutation at R98 and Y48 but does not harbour a mutation at position T99. In another embodiment, FimH harbours a mutation at position T99 and Y48 but does not harbour a mutation at position R98.

In even another embodiment, FimH harbours a R98A, or T99A, or Y48A mutation or any combination thereof. In one embodiment, FimH harbours either a R98A, or T99A, or Y48A mutation alone. In another embodiment, FimH harbours a triple mutation, i.e., R98A, T99A, and Y48A. In yet another embodiment, FimH harbours a R98A and T99A mutation but does not harbour a Y48A mutation. In even another embodiment, FimH harbour a R98A and Y48A mutation but does not harbour a T99A mutation. In another embodiment, FimH harbours a T99A and Y48A mutation but does not harbour a R98A mutation.

In yet another embodiment, FimH harbours a S17A, or I13A, or P12A mutation or any combination thereof. In one embodiment, FimH harbours either a S17A, or I13A, or P12A mutation alone. In another embodiment, FimH harbours a triple mutation, i.e., S17A, I13A, and P12A. In yet another embodiment, FimH harbours a S17A and I13A mutation but does not harbour a P12A mutation. In even another embodiment, FimH habours a S17A and P12A mutation but does not harbour a I31A mutation. In another embodiment, FimH habours a I13A and P12A mutation but does not harbour a S17A.

In yet another embodiment, FimH harbours a mutation at position S17, or 113, or P12 or any combination thereof. In one embodiment, FimH harbours either a mutation at position S17, or I13, or P12 alone. In another embodiment, FimH harbours a triple mutation, i.e., a mutation at position S17, 113, and P12. In yet another embodiment, FimH harbours a mutation at position S17 and 113 but does not harbour a mutation at position P12. In even another embodiment, FimH habours a mutation at position S17 and P12 but does not harbour a mutation at position 131. In another embodiment, FimH habours a mutation at position 113 and P12 but does not harbour a mutation at position S17.

In another embodiment, the drug or the compound is selected from the group consisting of D-mannose, small mannoside, CD14 antibody, CD14 binding molecule, CD14 inhibitor or an antibiotic. In one embodiment, the drug or compound is a CD14 binding molecule or CD14 inhibitor. In one embodiment, the CD14 binding molecule or CD14 inhibitor binds to e.g. Y48, and/or 152 and/or Y137 of FimH's tyrosine gate. Preferably, the CD14 binding molecule or CD14 inhibitor binds to e.g. Y48, and/or 152 and/or Y137 of FimH's tyrosine gate with high affinity. In another embodiment, the CD14 binding molecule or CD14 inhibitor binds to e.g. P1, and/or N46, and/or D47, and/or D54, and/or Q133, and/or D135, and/or D140, and/or F142 of FimH's mannose-binding pocket. Preferably, the CD14 binding molecule or CD14 inhibitor binds to e.g. P1, and/or N46, and/or D47, and/or D54, and/or Q133, and/or D135, and/or D140, and/or F142 of FimH's mannose-binding pocket with high affinity. In another embodiment, the CD14 inhibitor or CD14 binding molecule can also bind to any combination of the above disclosed amino acids, such as R98, any position(s) of FimH's tyrosine gate and any position(s) of the mannose-binding pocket. In a preferred embodiment, the CD14 binding molecule or CD14 inhibitor binds to R98, and/or T99, and/or Y48 of FimH. In a particularly preferred embodiment, the CD14 binding molecule or CD14 inhibitor binds to R98, and/or T99, and/or Y48 of FimH with high affinity. Alternatively, in another embodiment, the CD14 binding molecule or CD14 inhibitor binds with high affinity to any of S17, and/or 113, and/or P12, and/or D54, and/or F142, and/or N128N138, and/or V67, and/or D140, and/or Y137, and/or G16 of FimH. In a preferred embodiment, the CD14 binding molecule or CD14 inhibitor binds with high affinity to S17, and/or I13, and or P12 of FimH. Alternatively, in yet another embodiment, the CD14 binding molecule or CD14 inhibitor can also bind to e.g., D54, and/or F142, and/or N138, and/or V67, and/or D140, and/or Y137, and/or G16 of FimH. In another embodiment, the CD14 binding molecule or CD14 inhibitor can also bind to any combination of the above disclosed amino acids, such as S17 and any other position(s) of FimH. In yet another embodiment, the method is used in determining the potential of a drug for the treatment of urinary tract infections caused by type I piliated gram-negative bacteria.

In another embodiment, the method is used in determining the potential of a drug for the treatment of neonatal meningitis, inflammatory bowel diseases such as Crohn's disease, or ulcerative colitis, or gastrointestinal disorders or gastrointestinal diseases caused by type I piliated gram-negative bacteria.

In another aspect, the method is used to inform the selection of drugs in the treatment of urinary tract infections. This means that the method provides information which therapeutic agent (drug), e.g., CD14 antibody, CD14 inhibitor, CD14 binding molecule, antibiotic or any other agent (drug) potentially leads to a decreased FimH-CD14 interaction. Therefore, said therapeutic agent decreasing the FimH-CD14 is likely to be an efficacious compound with regards to its ability to counteract and ultimately, fight bacterial infections.

In yet another aspect, a CD14 antibody is used for the treatment of urinary tract infections. As defined above, a CD14 antibody can be any type of CD14 antibody blocking CD14. The antibody can be administered systemically (e.g., orally, parenterally, subcutaneously, intravenously, rectally, intramuscularly, intraperitoneally, intranasally, transdermally, or by inhalation or intracavitary installation), topically, or by application to mucosal membranes, such as the nose, throat, and bronchial tubes. Furthermore, the CD14 antibody can be administered, for example, thrice a week, twice a week, one a week to once every two, three, or four weeks. The CD14 antibody can be administered before the other treatment, concurrently with the treatment, after the treatment, or during remission of the infection. Alternatively, instead of a CD14 antibody, a CD14 inhibitor or CD14 binding molecule can be used for the treatment of urinary tract infections. Also, said CD14 inhibitor or CD14 binding molecule can be used for the treatment of any bacterial infection in which the inhibition or disruption of type I piliated gram-negative bacteria binding via FimH to CD14 is desirable. In one embodiment, the CD14 binding molecule or CD14 inhibitor used for the treatment of any bacterial infection in which the inhibition or disruption of type I piliated gram-negative bacteria binding via FimH to CD14 is desirable or used for the treatment of urinary tract infections in which the inhibition or disruption of type I piliated gram-negative bacteria binding via FimH to CD14 is desirable, binds to e.g., Y48, and/or I52 and/or Y137 of FimH's tyrosine gate. Preferably, said CD14 binding molecule or CD14 inhibitor binds to e.g. Y48, and/or I52 and/or Y137 of FimH's tyrosine gate with high affinity. In another embodiment, the CD14 binding molecule or CD14 inhibitor used for the treatment of any bacterial infection in which the inhibition or disruption of type I piliated gram-negative bacteria binding via FimH to CD14 is desirable or used for the treatment of urinary tract infections in which the inhibition or disruption of type I piliated gram-negative bacteria binding via FimH to CD14 is desirable binds to e.g., P1, and/or N46, and/or D47, and/or D54, and/or Q133, and/or D135, and/or D140, and/or F142 of FimH's mannose-binding pocket. Preferably, said CD14 binding molecule or CD14 inhibitor binds to e.g. P1, and/or N46, and/or D47, and/or D54, and/or Q133, and/or D135, and/or D140, and/or F142 of FimH's mannose-binding pocket with high affinity. In another embodiment, the CD14 inhibitor or CD14 binding molecule used for the treatment of any bacterial infection in which the inhibition or disruption of type I piliated gram-negative bacteria binding via FimH to CD14 is desirable or used for the treatment of urinary tract infections can also bind to any combination of the above disclosed amino acids, such as R98, any position(s) of FimH's tyrosine gate and any position(s) of the mannose-binding pocket. In a preferred embodiment, the CD14 binding molecule or CD14 inhibitor used for the treatment of any bacterial infection in which the inhibition or disruption of type I piliated gram-negative bacteria binding via FimH to CD14 is desirable or used for the treatment of urinary tract infections in which the inhibition or disruption of type I piliated gram-negative bacteria binding via FimH to CD14 is desirable, the CD14 binding molecule or CD14 inhibitor binds to R98, and/or T99, and/or Y48 of FimH. In a particularly preferred embodiment, said CD14 binding molecule or CD14 inhibitor binds to R98, and/or T99, and/or Y48 of FimH with high affinity. Alternatively, in another embodiment, the CD14 binding molecule or CD14 inhibitor used for the treatment of any bacterial infection in which the inhibition or disruption of type I piliated gram-negative bacteria binding via FimH to CD14 is desirable or used for the treatment of urinary tract infections in which the inhibition or disruption of type I piliated gram-negative bacteria binding via FimH to CD14 is desirable binds with high affinity to any of S17, and/or 113, and/or P12, and/or D54, and/or F142, and/or N138, and/or V67, and/or D140, and/or Y137, and/or G16 of FimH. In a preferred embodiment, the CD14 binding molecule or CD14 inhibitor used for the treatment of any bacterial infection in which the inhibition or disruption of type I piliated gram-negative bacteria binding via FimH to CD14 is desirable or used for the treatment of urinary tract infections in which the inhibition or disruption of type I piliated gram-negative bacteria binding via FimH to CD14 is desirable binds with high affinity to S17, and/or I13, and or P12 of FimH. Alternatively, in yet another embodiment, the CD14 binding molecule or CD14 inhibitor used for the treatment of any bacterial infection in which the inhibition or disruption of type I piliated gram-negative bacteria binding via FimH to CD14 is desirable or used for the treatment of urinary tract infections in which the inhibition or disruption of type I piliated gram-negative bacteria binding via FimH to CD14 is desirable can also bind to e.g., D54, and/or F142, and/or N138, and/or V67, and/or D140, and/or Y137, and/or G16 of FimH. In another embodiment, the CD14 binding molecule or CD14 inhibitor used for the treatment of any bacterial infection in which the inhibition or disruption of type I piliated gram-negative bacteria binding via FimH to CD14 is desirable or used for the treatment of urinary tract infections in which the inhibition or disruption of type I piliated gram-negative bacteria binding via FimH to CD14 is desirable can also bind to any combination of the above disclosed amino acids, such as S17 and any other position(s) of FimH. In yet another embodiment, a CD14 antibody can be combined with a CD14 inhibitor and/or a CD14 binding molecule.

In one embodiment, a combination of a CD14 antibody and antibiotics, or CD14 antibody and D-mannose, or CD14 antibody and mannoside, or CD14 antibody, antibiotics, and D-mannose or mannoside is used for the treatment of urinary tract infections. The combination of CD14 antibody, antibiotics and D-mannose can also be combined with mannoside. The combination of the agents can follow different treatment regimens, for example, the CD14 antibody can be administered weekly while the antibiotics can be administered daily or also multiple times per day. Similarly, the D-mannose or mannoside in combination with the CD14 antibody alone or in combination with the CD14 antibody and the antibiotics can be administered daily, once or twice daily, thrice or even more often daily. In another embodiment, the CD14 antibody, antibiotics, D-mannose and/or mannoside can be further combined with competitive inoculation (with e.g., less-pathogenic *E. coli*), vaccines (such as ExPEC4V), immunostimulants (e.g. Uro-Vaxom, Solco-Urovac or Uromune), intravescial glycosaminoglycans (e.g., hyaluronic acid, chondroitin sulfate), estrogens, methenamine hippurate, probiotics, NSAIDs (e.g., ibuprofen), cranberry products and propolis products. In another embodiment, a CD14 inhibitor and/or CD14 binding molecule is used in combination with antibiotics, or CD14 inhibitor and/or CD14 binding molecule and D-mannose, or CD14 inhibitor and/or CD14 binding molecule and mannoside is used for the treatment of urinary tract infections. In one embodiment, said CD14 inhibitor and/or CD14 binding molecule binds to e.g. Y48, and/or 152 and/or Y137 of FimH's tyrosine gate. Preferably, the CD14 binding molecule or CD14 inhibitor binds to e.g. Y48, and/or I52 and/or Y137 of FimH's tyrosine gate with high affinity. In another embodiment, the CD14 binding molecule or CD14 inhibitor binds to e.g. P1, and/or N46, and/or D47, and/or D54, and/or Q133, and/or D135, and/or D140, and/or F142 of FimH's mannose-binding pocket. Preferably, the CD14 binding molecule or CD14 inhibitor binds to e.g. P1, and/or N46, and/or D47, and/or D54, and/or Q133, and/or D135, and/or D140, and/or F142 of FimH's mannose-binding pocket with high affinity. In another embodiment, the CD14 inhibitor or CD14 binding molecule can also bind to any combination of the above disclosed amino acids, such as R98, any position(s) of FimH's tyrosine gate and any position(s) of the mannose-binding pocket. In a preferred embodiment, the CD14 binding molecule or CD14 inhibitor binds to R98, and/or T99, and/or Y48 of FimH. In a particularly preferred embodiment, the CD14 binding molecule or CD14 inhibitor binds to R98, and/or T99, and/or Y48 of FimH with high affinity. Alternatively, in another embodiment, the CD14 binding molecule or CD14 inhibitor binds with high affinity to any of S17, and/or 113, and/or P12, and/or D54, and/or F142, and/or N138, and/or V67, and/or D140, and/or Y137, and/or G16 of FimH. In a preferred embodiment, the CD14 binding molecule or CD14 inhibitor binds with high affinity to S17, and/or I13, and or P12 of FimH. Alternatively, in yet another embodiment, the CD14 binding molecule or CD14 inhibitor can also bind to e.g., D54, and/or F142, and/or N138, and/or V67, and/or D140, and/or Y137, and/or G16 of FimH. In another embodiment, the CD14 binding molecule or CD14 inhibitor can also bind to any combination of the above disclosed amino acids, such as S17 and any other position(s) of FimH. In yet another embodiment, the CD14 inhibitor and/or CD14 binding molecule, antibiotics, D-mannose and/or mannoside can be further combined with competitive inoculation (with e.g., less-pathogenic *E. coli*), vaccines (such as ExPEC4V), immunostimulants (e.g. Uro-Vaxom, Solco-Urovac or Uromune), intravescial glycosaminoglycans (e.g., hyaluronic acid, chondroitin sulfate), estrogens, methenamine hippurate, probiotics, NSAIDs (e.g., ibuprofen), cranberry products and propolis products.

In another embodiment, a CD14 antibody is used for the treatment of persistent and/or recurrent urinary tract infections. As defined above, a CD14 antibody can be any type of CD14 antibody that targets and therefore, binds to CD14. In a particularly preferred embodiment, the CD14 antibody is a recombinant monoclonal chimeric antibody. In a particularly preferred embodiment, the CD14 antibody is Atibuclimab. Atibuclimab can be administered systemically (e.g., orally, parenterally, subcutaneously, intravenously, rectally, intramuscularly, intraperitoneally, intranasally, transdermally, or by inhalation or intracavitary installation), topically, or by application to mucosal membranes, such as the nose, throat, and bronchial tubes. Furthermore, Atibuclimab can be administered, for example, thrice a week, twice a week, once a week to once every two, three, or four weeks. Atibuclimab can be administered before the other treatment, concurrently with the treatment, after the treatment, or during remission of the infection.

In one embodiment, the CD14 antibody for use in the treatment of urinary tract infections, or persistent and/or recurrent urinary tract infections is Atibuclimab. Atibuclimab can be administered systemically (e.g., orally, parenterally, subcutaneously, intravenously, rectally, intramuscularly, intraperitoneally, intranasally, transdermally, or by inhalation or intracavitary installation), topically, or by application to mucosal membranes, such as the nose, throat, and bronchial tubes. Furthermore, Atibuclimab can be administered, for example, thrice a week, twice a week, once a week to once every two, three, or four weeks. Atibuclimab can be administered before any other treatment, concurrently with the treatment, after the treatment, or during remission of the infection.

### EXAMPLES

### Example 1- mutants

To verify how crucial the predicted amino acids are for binding to CD14, we introduced single amino acid mutations into the three most important amino acids. We exchanged amino acids R98 (binding energy -7.23 kcal/mol), T99 (binding energy -4.92 kcal/mol) and Y48 (binding energy -4.29 kcal/mol) individually to alanine (A) and all three at the same time creating a triple mutant (Figure 1). Surprisingly, all four FimH mutants are still able to bind to CD14-coupled beads and only the triple mutant showed some decreased binding, although nowhere close to the FimH deletion mutant (Figure 2).

Since especially Y48 but also other amino acids with weaker binding energy are located in the mannose binding pocket, we were wondering if FimH antagonists, such as D-mannose or the small mannoside M4284, could inhibit binding of FimH to CD14. Additionally, we tested a blocking CD14 antibody (M14-23) for its ability to inhibit binding of FimH to CD14. The suggested upper daily limit of orally applied D-mannose to treat UTI of 9 g leads to blood mannose levels of roughly 175 µM. We therefore tested 175 µM and 1 mM of D-mannose. Interestingly, 175 µM D-mannose was not sufficient to block binding of UPEC ON bacteria to CD14, whereas 1 mM D-mannose and the small mannoside M4284 at 10 µM were able to inhibit binding of UPEC ON bacteria to CD14-coupled beads in the bead-binding assay (Figure 3). Interestingly, the blocking CD14 antibody was not able to block binding of the bacteria to the beads fully, indicating that the binding site for the antibody on CD14 is different from the FimH binding site. Our analysis reveals that the amino acids in FimH involved in binding to CD14 are highly conserved among different *E. coli* strains making them a potential target for drug treatment or drug design (Figure 1).

Moreover, FimH antagonists, as well as a blocking CD14 antibody, can be used to block binding of type I piliated UPEC to CD14. The small mannoside M4284 displaying strongly increased binding to FimH or the blocking CD14 antibody could be of greater interest in clinical practice to block the adverse effect of type I piliated UPEC on dendritic cells.

### Example 2- bead assay

5 µl of 10 µm bead slurry (PureProteom Protein A magnetic beads, Merck Millipore, Darmstadt, Germany) were used per reaction. Beads were washed 3x with PBS containing 0.005 % Tween^{®}20 (PBS-T). Beads were collected using a magnetic stand. 5 µg CD14-Fc recombinant protein in total of 10µl of PBS-T was coupled to the beads for one hour at 4 °C with rotation. After washing beads, 100µl of bacteria grown to mid-exponential phase in CAA M9 glycerol medium (∼1*10⁷ cells) were incubated in presence of Tween^{®}20 with the CD14-coupled beads for one hour at 4 °C with rotation. At the same time, D-mannose at 175 µM or 1 mM, the small mannoside M4284, the blocking CD14 antibody M14-23, tetracycline and the tetracycline-analog anhydrotetracycline were added, respectively. After washing again, this time with M9 glycerol medium containing Tween^{®}20, bead-bound bacteria were fixed with 0.5 % paraformaldehyde in M9 buffer for 10 min at 4 °C. Alternatively, if a S2 flow cytometer and microscope are available, no fixation with paraformaldehyde is required and the fixation step can be omitted. Similarly, if non-pathogenic bacteria are used, no fixation with paraformaldehyde is required and the fixation step can be omitted, too. For the subsequent flow cytometry analysis, samples were diluted in M9 buffer and analyzed on FACS Canto II (BD, Franklin Lakes, NJ, USA). 10,000 events gated on FSC-A and SSC-A to exclude debris were recorded at medium flow rate and data were analyzed using FlowJo^{™} software (BD, Franklin Lakes, NJ, USA). For this assay, bacteria expressing a chromosomally encoded fluorescence maker were used. However, we confirmed that also external labeling of bacteria, for example with thiazole orange, works.

Since some of the identified amino acids on FimH are located in the mannose binding pocket, we were wondering if FimH antagonists, such as D-mannose or the small mannoside M4284, could inhibit binding of bacteria expressing FimH (ON) to CD14. Additionally, we tested a blocking CD14 antibody (M14-23) for its ability to inhibit binding of FimH to CD14. Also, with the help of a protein-protein docking model of FimH and CD14, we also identified tetracycline like drugs as a potential inhibitor as those that bind in the same region to CD14 as FimH does. We therefore also analyzed the inhibitory potential of tetracycline and the non-bacteriostatic analog anhydrotetracycline. A bacterial mutant not expressing FimH was used as a negative control (ON *ΔfimH*).

The suggested upper daily limit of orally applied D-mannose to treat UTI of 9 g leads to blood mannose levels of roughly 175 µM. We therefore tested 175 µM and 1 mM of D-mannose. Interestingly, 175 µM D-mannose was not sufficient to block binding of ON bacteria to CD14, whereas 1 mM D-mannose and the small drug M4284 at 10 µM were able to inhibit binding of ON bacteria to CD14-coupled beads (Figure 3). Interestingly, the blocking CD14 antibody was not able to block binding of the bacteria to the beads fully, indicating that the binding site for the antibody on CD14 is different from the FimH binding site. Also, sub-minimal inhibitory concentrations of tetracycline or the tetracycline-analog anhydrotetracycline were not able to prevent binding of ON bacteria to CD14-coupled beads.

### Example 3-adhesion assay

Hox8b-derived murine dendritic cells prepared as per the method published by Leithner et al. 2018 in the European Journal of Immunology were seeded at a density of 1-2*10⁵ cells/ml in R10H20 medium using black 24-well tissue-treated dishes. The dendritic cells were stimulated with bacteria at a multiplicity of infection of 10 (10 bacteria per 1 dendritic cell). At the same time, D-mannose at 175 µM or 1 mM, the small mannoside M4284, the blocking CD14 antibody M14-23, tetracycline and the tetracycline-analog anhydrotetracycline were added, respectively. One hour post infection, 10µg/ml gentamicin was added to prevent extracellular growth of bacteria. 18 to 20 hours post infection, the adhesion assay was performed by removing non-adherent dendritic cells and washing adherent cells twice with 500 µl PBS. Adherent cells were stained with Hoechst 3342 (NucBlue reagent, 2 drops/ml) in R10H20 medium for 30 min at 37 °C. Cells were washed again and 1ml Live Cell Imaging solution (140 mM NaCl, 2.5 mM KCI, 1.8 mM CaCl₂, 1.0 mM MgCl₂, 20mM HEPES, pH 7.4) was added to the wells. Multiple brightfield images were taken from randomly chosen fields of view at 4x magnification. Fluorescence emitted from a bulk or population of adherent dendritic cells was measured with a Synergy H1 plate reader (BioTeK, Winooski, VT, USA). 50 data points per well were read, the bottom of the dished were read without a lid at an excitation of 490 nm and an emission of 520 nm.

ON bacteria expressing FimH increase adhesion of dendritic cells to cell culture treated plastic in the presence of serum proteins, as compared to ON bacteria lacking the FimH protein. Low concentrations of D-mannose (175 µM) did not decrease adhesion of dendritic cells, whereas increased concentrations of D-mannose (1 mM) and the small mannoside M4284 were partially able to do so (Figure 4 and 5). Surprisingly, the blocking CD14 antibody M14-23, which did not block biding of the bacteria to CD14 in the bead assay, was able to clearly reduce adhesion of dendritic cells. In stark contrast, tetracycline and the tetracycline-analog anhydrotetracycline significantly increased adhesion of dendritic cells. This means that both tetracycline and anhydrotetracycline would not be a suitable treatment for urinary tract infections in this particular example.

## Claims

1. A method for testing a drug for the treatment of bacterial infections, wherein the method is **characterized by** determining the potential of the drug to inhibit binding of type I piliated gram-negative bacteria to CD14 *in vitro.*

2. The method according to claim 1, wherein the type I piliated gram-negative bacteria express FimH.

3. The method according to claim 1 or 2, wherein the method comprises the steps of:
a) coupling magnetic beads to a CD14 protein,
b) providing type I piliated gram-negative bacteria having a fluorescent marker stably integrated in their DNA or, alternatively, visualizing the DNA by a fluorescent marker which is added after step f),
c) adding said type I piliated gram-negative bacteria expressing FimH to the CD14-coupled magnetic beads,
d) incubating said type I piliated gram-negative bacteria with the CD14-coupled magnetic beads and adding a drug or a compound,
e) removing any unbound bacteria from the beads,
f) optionally fixing the bound bacteria with paraformaldehyde,
g) analyzing the type I piliated gram-negative bacteria bound to the CD14-coupled magnetic beads by fluorescence microscopy or flow cytometry, wherein a decrease in the at least one fluorescent marker in the presence of the drug or the compound indicates that the drug or the compound inhibits binding of FimH to CD14.

4. The method of any one of claims 1 to 3, wherein D-mannose, mannoside and/or a CD14 antibody inhibit binding of gram-negative bacteria expressing FimH to CD14 coupled magnetic beads.

5. The method according to claim 1 or 2, wherein the method comprises the steps of:
a) providing dendritic cells,
b) infecting said dendritic cells with type I piliated gram-negative bacteria expressing FimH,
c) adding a drug or a compound to the dendritic cells,
d) adding an antibiotic to kill the bacteria that have not been engulfed by said dendritic cells,
e) removing non-adherent dendritic cells after a defined time period, thereby
f) obtaining infected adherent dendritic cells only,
g) adding a fluorescent dye to said infected adherent dendritic cells,
h) analyzing the dendritic cells by a fluorescence plate reader or fluorescence microscopy, wherein a decrease in the at least one fluorescent signal in the presence of the drug or the compound indicates that the drug or the compound decreases adhesion of said infected dendritic cells.

6. The method of claim 5, wherein the dendritic cells are infected murine dendritic cells.

7. The method of claim 5 or 6, wherein the drug or compound leads to decreased adhesion of the dendritic cells compared to untreated dendritic cells.

8. The method of any one of claims 1 to 7, wherein the type I piliated gram-negative bacteria are selected from *Escherichia coli* and/or *Klebsiella pneumoniae, or Salmonella spp.*

9. The method of any one of claims 2 to 8, wherein FimH harbors a R98A, or T99A, or Y48A mutation or any combination thereof.

10. The method according to any one of claims 1 to 9, wherein the drug or the compound is selected from the group consisting of D-mannose, small mannoside, CD14 antibody, CD14 binding molecule, CD14 inhibitor or an antibiotic.

11. The method according to any one of claims 1 to 10, wherein the method is used in determining the potential of a drug for the treatment of urinary tract infections caused by type I piliated gram-negative bacteria.

12. The method according to any one of claims 1 to 11, wherein the method is used in determining the potential of a drug for the treatment of neonatal meningitis, inflammatory bowel diseases such as Crohn's disease, or ulcerative colitis, or gastrointestinal disorders or gastrointestinal diseases caused by type I piliated gram-negative bacteria.

13. Use of the method of any one of claims 1 to 12, wherein the use of the method informs the selection of drugs in the treatment of urinary tract infections.

14. A CD14-antibody for use in the treatment of urinary tract infections.

15. A combination of a CD14 antibody and antibiotics, or CD14 antibody and D-mannose, or CD14 antibody and mannoside, or CD14 antibody, antibiotics and D-mannose or mannoside for use in the treatment of urinary tract infections.

16. A CD14-antibody for use in the treatment of persistent and/or recurrent urinary tract infections.

17. A CD14-antibody for the use according to any one of claims 14 to 16, wherein the CD14 antibody is Atibuclimab.
